# EUROPEAN PATENT APPLICATION

(11) **EP 3 037 095 A2**
(43) Date of publication of application: **29.06.2016**
(21) Application number: 16152228.9
(22) Date of filing: 08.11.2007
(51) Int. Cl.: A61K 31/341, A61K 31/70, A61P 3/00, A61P 3/10, A61K 31/351, A61K 31/155, A61K 45/06

(54) **COMBINATION THERAPY WITH SGLT-2 INHIBITORS AND THEIR PHARMACEUTICAL COMPOSITIONS**

(30) Priority: 09.11.2006 US 865099 P
(62) Divisional of application: 07822331.0
(71) Applicant: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: MANUCHEHRI, Alireza, Berkshire, RG40 1PE (GB); DUGI, Klaus, 55216 INGELHEIM AM RHEIN (DE); EICKELMANN, Peter, 55216 INGELHEIM AM RHEIN (DE); THOMAS, Leo, 55216 INGELHEIM AM RHEIN (DE)
(74) Representative: Simon, Elke Anna Maria

(57) **Abstract**

The present invention is directed to a pharmaceutical composition comprised of one or more SGLT-2 inhibitor compound(s) in combination with one or more therapeutic agents which is suitable for the treatment of metabolic disorders including type 1 diabetes mellitus, type 2 diabetes mellitus, impaired glucose tolerance, hyperglycemia, postprandial hyperglycemia, overweight, obesity, including class I obesity, class II obesity, class III obesity, visceral obesity and abdominal obesity, and metabolic syndrome.

## Description

### TECHNICAL FIELD OF THE INVENTION

The present invention relates to sodium dependent glucose co-transporter 2 (SGLT-2) Inhibitors in combination with other active ingredients, their pharmaceutical compositions, processes for preparing them and their use in the treatment of metabolic diseases such as type 1 diabetes mellitus, type 2 diabetes mellitus, impaired glucose tolerance and hyperglycemia and related diseases.

### BACKGROUND OF THE INVENTION

Metabolic diseases and associated disorders are becoming increasingly prevalent. In particular, type 2 diabetes mellitus (T2DM) a metabolic disease, is an increasingly prevalent disease that due to a high frequency of complications leads to a significant reduction of life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

Most orally available antidiabetic drugs that are on the market or in the late-stage of clinical development, predominantly target one of the following modes of action:
- Reduction in endogenous glucose production by the liver: *biguanides (e.g. metformin)*
- Promoting insulin secretion from β-cells with residual function: *sulfonylureas, meglitinides*
- Reduction in peripheral insulin resistance: *thiazolidinediones* (TZD)
- Delaying glucose absorption from the gut: *α-glucosidase inhibitors,*
- Promoting glucose-dependent insulin secretion, suppressing elevated glucagon levels, and delaying gastric emptying: *incretin mimetics (e.g. exenatide), amaylin analogues (e.g. pramlintide), dipeptidiyl peptidase IV inhibitors (e.g. sitagliptin)*

Despite targeting various modes of action, the first three substance classes are remarkably similar in efficacy with regard to glycaemic control (∼1.0-1.5% absolute reduction of HbA1c, if the mean baseline is ≥ 8.0%). However, as their modes of action differ, they are suitable for combination treatment.

The efficacy of specific inhibitors of glucose absorption (intestinal α-glucosidase inhibitors, prototype *acarbose*) with regard to glycemic control is less than that of the aforementioned other mechanisms of action.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the proximal tubuli along the sodium gradient ⁽¹⁾. There are at least 3 SGLT isoforms that differ in their expression pattern as well as in their physico-chemical properties ⁽²⁾. SGLT2 is exclusively expressed in the kidney ⁽³⁾, whereas SGLT1 is expressed additionally in other tissues like intestine, colon, skeletal and cardiac muscle ^{(4;5)}. SGLT3 has been found to be a glucose sensor in interstitial cells of the intestine without any transport function ⁽⁶⁾. Potentially, other related, but not yet characterized genes, may contribute further to renal glucose reuptake ^{(7,8,9)}. Under normoglycemia, glucose is completely reabsorbed by SGLTs in the kidney, whereas the reuptake capacity of the kidney is saturated at glucose concentrations higher than 10mM, resulting in glucosuria ("diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentrations and to glucosuria ^{(10;11)}.
(1) Wright, E.M. (2001) Am. J. Renal Physiol. 280, F10-F18;
(2) Wright, E.M. et al. (2004) Pflugers Arch. 447(5):510-8;
(3) You, G. et al. (1995) J. Biol. Chem. 270 (49) 29365-29371;
(4) Pajor AM, Wright EM (1992) J Biol. Chem. 267(6):3557-3560;
(5) Zhou, L. et al. (2003) J. Cell. Biochem. 90:339-346;
(6) Diez-Sampedro, A. et al. (2003) Proc. Natl. Acad. Sci. USA 100(20), 11753-11758;
(7) Tabatabai, N.M. (2003) Kidney Int. 64, 1320-1330;
(8) Curtis, R.A.J. (2003) US Patent Appl. 2003/0054453;
(9) Bruss,M. and Bonisch,H. (2001) Cloning and functional characterization of a new human sugar transporter in kidney (Genbank Acc. No. AJ305237);
(10) Rossetti, L. Et al. (987) J. Clin. Invest. 79, 1510-1515;
(11) Gouvea, W.L. (1989) Kidney Int. 35(4):1041-1048.

Type 2 diabetes is an increasingly prevalent disease that due to a high frequency of complications leads to a significant reduction of life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements and monitoring to adjust the dose of their insulin.

The UKPDS (United Kingdom Prospective Diabetes Study) demonstrated that intensive treatment with metformin, sulfonylureas or insulin resulted in only a limited improvement of glycemic control (difference in HbA1c ∼0.9%). In addition, even in patients within the intensive treatment arm glycemic control deteriorated significantly over time and this was attributed to deterioration of β-cell function. Importantly, intensive treatment was not associated with a significant reduction in macrovascular complications, i.e. cardiovascular events.

US 20050209166 (Ser. No. 11/080,150) describes novel glucopyranosyl-substituted phenyl derivatives. The glucopyranosyl-substituted phenyl derivatives are suitable for the treatment of metabolic diseases.

Glucopyranosyloxy- substituted aromatic groups and the preparation thereof and their possible activity as SGLT-2 inhibitors are known from published International applications WO 98/31697, WO 01/27128, WO 02/083066, WO 03/099836, WO 2004/063209, WO 2004/080990, WO 2004/013118, WO 2004/052902, WO 2004/052903 and US application US 2003/0114390 the contents of which are incorporated herein.

Therefore there is an unmet medical need for methods, medicaments and pharmaceutical compositions with good efficacy with regard to glycemic control, with regard to disease-modifying properties and with regard to reduction of cardiovascular morbidity and mortality.

### SUMMARY OF THE INVENTION

The present invention is directed to a pharmaceutical composition comprised of one or more SGLT-2 inhibitor compound(s) in combination with one or more therapeutic agents which is suitable for the treatment of metabolic disorders.

More particularly, the present invention is directed to a pharmaceutical composition wherein the SGLT-2 inhibitor compound is an Active Ingredient A compound as described herein in combination with an Active Ingredient B compound as described herein for the treatment of metabolic disorders.

In another embodiment of the invention the metabolic disorder is selected from the group consisting of type 1 diabetes mellitus, type 2 diabetes mellitus, impaired glucose tolerance, hyperglycemia, postprandial hyperglycemia, overweight, obesity, including class I obesity, class II obesity, class III obesity, visceral obesity and abdominal obesity, and the metabolic syndrome.

The Active Ingredients can be administered in a single pharmaceutical composition or administered individually. In one embodiment, a pharmaceutical composition according to the invention is suitable for combined or simultaneous or sequential use of the one or more SGLT-2 inhibitor compound(s) and the one or more second therapeutic agent(s). In one embodiment, the one or more SGLT-2 inhibitor compound(s) and the one or more second therapeutic agent(s) are present in a single dosage form. In another embodiment, the one or more SGLT-2 inhibitor compound(s) and the one or more second therapeutic agent(s) are present each in a separate dosage form.

The present invention is also directed to a composition comprising a Glucopyranosyl-substituted phenyl derivatives selected from the group of compounds 1-17:
(1) 1-chloro-2-(4-cyclopentyloxybenzyl)-4-(B-D-glucopyranos-1-yl)-benzene
(2) 1-chloro-4-(ß-D-glucopyranos-1-yl)-2-[4-(*(R)*-tetrahydrofuran-3-yloxy)-benzyl]-benzene
(3) 1-chloro-4-(ß-D-glucopyranos-1-yl)-2-[4-(*(S)*-tetrahydrofuran-3-yloxy)-benzyl]-benzene
(4) 1-chloro-4-(ß-D-glucopyranos-1-yl)-2-[4-(tetrahydrofuran-2-on-3-yloxy)-benzyl]-benzene
(5) 1-chloro-4-(ß-D-glucopyranos-1-yl)-2-(4-cyclobutyloxy-benzyl)-benzene
(6) 1-chloro-4-(ß-D-glucopyranos-1-yl)-2-(4-cyclohexyloxy-benzyl)-benzene
(7) 1-chloro-4-(B-D-glucopyranos-1-yl)-2-[4-(tetrahydropyran-4-yloxy)-benzyl]-benzene
(8) 1-chloro-4-(B-D-glucopyranos-1-yl)-2-[4-(1-acetyl-piperidin-4-yloxy)-benzyl]-benzene
(10) 1-(B-D-Glucopyranos-1-yl)-4-methyl-3-[4-(tetrahydrofuran-3-yloxy)-benzyl]-benzene
(11) 1-(ß-D-Glucopyranos-1-yl)-4-methyl-3-[4-(2-trimethylsilyl-ethyl)-benzyl]-benzene
(12) 1-chloro-4-(ß-D-glucopyranos-1-yl)-2-(4-ethynyl-benzyl)-benzene
(13) 1-chloro-4-(ß-D-glucopyranos-1-yl)-2-[4-(piperidin-4-yloxy)-benzyl]-benzene
(14) 1-fluoro-4-(ß-D-glucopyranos-1-yl)-2-(4-ethynyl-benzyl)-benzene
(15) 1-(ß-D-glucopyranos-1-yl)-3-(4-ethynyl-benzyl)-benzene
(16) 1-ethynyl-4-(ß-D-glucopyranos-1-yl)-2-(4-ethoxy-benzyl)-benzene
(17) 1-methoxy-4-(ß-D-glucopyranos-1-yl)-2-(4-ethynyl-benzyl)-benzene
or a prodrug thereof wherein one or more hydroxyl groups of the β-D-glucopyranosyl group are acylated with groups selected from (C₁₋₃-alkyl)carbonyl, (C₁₋₆-alkyl)oxycarbonyl, phenylcarbonyl, phenyl-(C₁₋₃-alkyl)-carbonyl, phenyloxycarbonyl and phenyl-(C₁₋₃-alkyl)-oxycarbonyl, or a pharmaceutically acceptable salt thereof;
in combination with at least one second therapeutic agent which is suitable in the treatment or prevention of one or more conditions selected from type 1 diabetes mellitus, type 2 diabetes mellitus, impaired glucose tolerance and hyperglycemia.

The present invention is also directed to methods for treating and prevention metabolic disorders using the pharmaceutical compositions of the invention.

The present invention is also directed to a method of treating a metabolic disease selected from the group consisting of type 1 diabetes mellitus, type 2 diabetes mellitus, impaired glucose tolerance, hyperglycemia, postprandial hyperglycemia, overweight, obesity, including class I obesity, class II obesity, class III obesity, visceral obesity and abdominal obesity, and metabolic syndrome said method comprised of the step of administering to a patient in need therof a combination of an Active Ingredient A with an Active Ingredient B in a therapeutic amount in a patient in need thereof.

The present invention is also directed to the use of a pharmaceutical composition according to the present invention for the manufacture of a medicament for a therapeutic or preventive method as disclosed herein. In one aspect, the present invention is also directed to the use of a glucopyranosyl-substituted benzene derivative disclosed herein for the manufacture of a medicament for use in a method disclosed herein. In another aspect, the present invention is also directed to use of a second therapeutic agent disclosed herein for the manufacture of a medicament for use in a method disclosed herein.

### DETAILED DESCRIPTION OF THE PREFERRED EMBODIMENTS

Due to the low affinity of SGLT-2 towards glucose, inhibition of this transporter will be effective in hyperglycemic states, thereby limiting the danger of hypoglycemia in treated patients, since this is prevented by the remaining (high affinity) activity of SGLT-1. This is supported by the fact that patients with a homozygous defect in the SGLT-2 gene show renal glucosuria, but have normal plasma glucose levels and are otherwise healthy.

Therefore, SGLT-2 inhibitors, due to their unique mode of action, are expected to lower blood glucose and HbAlc with a low associated risk of hypoglycaemia. Furthermore, given that the excretion of glucose in urine as a result of SGLT-2 inhibition may cause mild diuresis (as seen in individuals with congenital SGLT-2 deficiency), the use of Glucopyranosyl-substituted phenyl derivatives in combination with insulin and TZD, drugs that are known to cause fluid retention, may be of special interest. This potential attribute may be particularly important in the management of diabetic patients in the acute phase of myocardial infarction. Such patients are susceptible to acute heart failure secondary to fluid retention as a result of the insulin therapy which is currently recommended for these patients in the treatment guidelines.

A beneficial therapeutic effect, particularly an additive or over-additive effect or an overall reduction of side effects of therapy, is desirable in the treatment of metabolic diseases and particularly diabetes mellitus, in patients whose glycemic control is poor or suboptimal on one antidiabetic agent, those with the metabolic syndrome, prediabetes/impaired glucose tolerance, obesity, and metabolic conditions linked to obesity and insulin resistance such as polycystic ovarian syndrome (PCOS).

Administration of one or more of the Active Ingredients A and an Active Ingredient B can have an additive or over-additive effect of the pharmaceutical combinations according and provide for dose reduction, side-effect reduction and/or interval extension when compared to the individual Active Ingredient A and Active Ingredient B used in monotherapy in the usual way. The effects mentioned above are observed both when the two active substances are administered simultaneously in a single active substance formulation and when they are administered successively in separate formulations. In the case of Active Ingredient B being an injectable, especially a biological agent, other benefits of adding Active Ingredient A may be seen. For example, cost reduction by way of interval and/or dose reduction.

### Definitions

The term "obesity" is defined as the condition wherein the individual has a BMI equal to or greater than 30 kg/m². According to a WHO definition the term obesity may be categorized as follows: the term "class I obesity" is the condition wherein the BMI is equal to or greater than 30 kg/m² but lower than 35 kg/m²; the term "class II obesity" is the condition wherein the BMI is equal to or greater than 35 kg/m² but lower than 40 kg/m²; the term "class III obesity" is the condition wherein the BMI is equal to or greater than 40 kg/m².

The term "**hyperglycemia**" is defined as the condition in which a subject has a fasting blood glucose concentration above the normal range, greater than 110 mg/dL (6.11 mmoI/L). The word "fasting" has the usual meaning as a medical term.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycemic-hyperinsulinemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are the so called minimal models in which, during an intravenous glucose tolerance test, the insulin and glucose concentrations in the blood are measured at fixed time intervals and from these the insulin resistance is calculated. In this method it is not possible to distinguish between hepatic and peripheral insulin resistance.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, coronary heart disease (CHD).

The term **"HbA1c"** refers to the product of a non-enzymatic glycation of the haemoglobin beta chain. Its determination is well known to one skilled in the art. In monitoring the treatment of diabetes mellitus the HbAlc value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients, whose HbAlc value is consistently well adjusted by intensive diabetes treatment (i.e. < 6.5 %, preferably < 6.0 % of the total haemoglobin in the sample), are significantly better protected against diabetic microangiopathy. For example metformin on its own achieves an average improvement in the HbAlc value in the diabetic of the order of 1.0 - 1.5 %. This reduction of the HbA1C value is not sufficient in all diabetics to achieve the desired target range of < 6.5 %, preferably < 6.0 %, HbA1c.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:
1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 110 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

### A) Active Ingredient A Compounds which may be used according to the invention

Active Ingredient A compounds are typically SGLT-2 inhibitor compounds. Suitable Active Ingredient A compounds include compounds chosen from those disclosed in US Patent application US20050209166 the contents of which is incorporated herein by reference in its entirety. The pharmaceutical compositions according to the invention can be SGLT-2 inhibitors compounds of formula I: 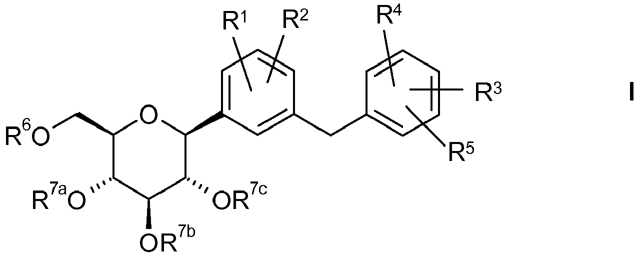 wherein
- R¹: is selected from the definitions of the group A and
if R³ is selected from the definitions of the group B, R¹ may additionally also be selected from the meanings hydrogen, fluorine, chlorine, bromine, iodine, C₁₋₄-alkyl, C₂₋₄-alkenyl-C₁₋₄-alkyl, C₂₋₄-alkynyl-C₁₋₄-alkyl, C₂₋₄-alkenyl-C₁₋₄-alkoxy, C₂₋₄-alkynyl-C₁₋₄-alkoxy, C₃₋₇-cycloalkyl-C₁₋₄-alkyl, C₅₋₇-cycloalkenyl-C₁₋₄-alkyl, a methyl group substituted by 1 to 3 fluorine atoms, an ethyl group substituted by 1 to 5 fluorine atoms, C₁₋₄-alkoxy, a methoxy group substituted by 1 to 3 fluorine atoms, an ethoxy group substituted by 1 to 5 fluorine atoms, a C₁₋₄-alkyl group substituted by a hydroxy or C₁₋₃-alkoxy group, a C₂₋₄-alkoxy group substituted by a hydroxy or C₁₋₃-alkoxy group, C₃₋₆-cycloalkyl-C₁₋₃-alkoxy or hydroxy,
while in the above-mentioned cycloalkyl and cycloalkenyl rings one or two methylene groups may be replaced independently of one another by O or CO, and
- R²: denotes hydrogen, fluorine, chlorine, bromine, hydroxy, C₁₋₄-alkyl, C₁₋₄-alkoxy, cyano or nitro, while the alkyl or alkoxy group may be mono- or polysubstituted by fluorine, and
- R³: is selected from the definitions of the group B and
if R¹ is selected from the definitions of the group A, R³ may additionally also be selected from the meanings hydrogen, fluorine, chlorine, bromine, iodine, C₁₋₆-alkyl, C₂₋₄-alkenyl-C₁₋₄-alkyl, C₂₋₄-alkynyl-C₁₋₄-alkyl, C₂₋₄-alkenyl-C₁₋₄-alkoxy, C₂₋₄-alkynyl-C₁₋₄-alkoxy, C₃₋₇-cycloalkyl, C₅₋₇-cycloalkenyl, C₃₋₇-cycloalkyl-C₁₋₄-alkyl, C₅₋₇-cycloalkenyl-C₁₋₄-alkyl, C₃₋₆-cycloalkylidenmethyl, hydroxy, C₁₋₆-alkoxy, C₃₋₆-cycloalkyl-C₁₋₃-alkoxy, aryl, aryl-C₁₋₃-alkyl, heteroaryl, heteroaryl-C₁₋₃-alkyl, aryloxy, aryl-C₁₋₃-alkyl-oxy, a methyl or methoxy group substituted by 1 to 3 fluorine atoms, a C₂₋₄-alkyl or C₂₋₄-alkoxy group substituted by 1 to 5 fluorine atoms, a C₁₋₄-alkyl group substituted by a cyano group, a C₁₋₄-alkyl group substituted by a hydroxy or C₁₋₃-alkyloxy group, cyano, carboxy, C₁₋₃-alkoxycarbonyl, aminocarbonyl, (C₁₋₃-alkylamino)carbonyl, di-(C₁₋₃-alkyl)aminocarbonyl, pyrrolidin-1-ylcarbonyl, piperidin-1-ylcarbonyl, morpholin-4-ylcarbonyl, piperazin-1-yl-carbonyl, 4-(C₁₋₃-alkyl)-piperazin-1-ylcarbonyl, (C₁₋₄-alkyl)carbonylamino, C₁₋₄-alkylsulphonylamino, C₁₋₄-alkylsulphanyl, C₁₋₄-alkylsulphinyl, C₁₋₄-alkylsulphonyl, arylsulphonylamino, aryl-C₁₋₃-alkylsulphonylamino or arylsulphonyl,
- R⁴, R⁵: independently of one another denote hydrogen, fluorine, chlorine, bromine, iodine, cyano, nitro, C₁₋₃-alkyl, C₁₋₃-alkoxy, methyl or methoxy substituted by 1 to 3 fluorine atoms,
- A: denotes C₂₋₆-alkyn-1-yl, C₂₋₆-alken-1-yl, C₃₋₇-cycloalkyl, C₅₋₇-cycloalkenyl, aryl, heteroaryl, C₁₋₄-alkylcarbonyl, arylcarbonyl, heteroarylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, arylaminocarbonyl, heteroarylaminocarbonyl, C₁₋₄-alkoxycarbonyl, aryl-C₁₋₃-alkoxycarbonyl, heteroaryl-C₁₋₃-alkoxycarbonyl, amino, C₁₋₄-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, C₁₋₄-alkylcarbonylamino, arylcarbonylamino, heteroarylcarbonylamino, C₃₋₇-cycloalkyloxy, C₅₋₇-cycloalkenyloxy, aryloxy, heteroaryloxy, C₁₋₄-alkylsulphinyl, C₁₋₄-alkyl-sulphonyl, C₃₋₇-cycloalkylsulphanyl, C₃₋₇-cycloalkylsulphinyl, C₃₋₇-cycloalkylsulphonyl, C₅₋₇-cycloalkenylsulphanyl, C₅₋₇-cycloalkenylsulphinyl, C₅₋₇-cycloalkenylsulphonyl, arylsulphanyl, arylsulphinyl, arylsulphonyl, heteroarylsulphanyl, heteroarylsulphinyl, heteroarylsulphonyl, cyano or nitro,
while the above-mentioned alkynyl and alkenyl groups may be mono- or polysubstituted by fluorine or chlorine, and
the above-mentioned alkynyl and alkenyl groups may be mono- or disubstituted by identical or different groups L1, and
the above-mentioned cycloalkyl and cycloalkenylrings independently of one another may be mono- or disubstituted by substituents selected from fluorine and C₁₋₃-alkyl, and
in the above-mentioned cycloalkyl and cycloalkenyl rings one or two methylene groups may be replaced independently of one another by O, S, CO, SO, SO₂ or NR^{N},
- B: denotes tri-(C₁₋₄-alkyl)silyl-C₁₋₆-alkyl, C₂₋₆-alkyn-1-yl, C₂₋₆-alken-1-yl, amino, C₁₋₃-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, arylcarbonylamino, heteroarylcarbonylamino, nitro, C₃₋₁₀-cycloalkyloxy, C₅₋₁₀-cycloalkenyloxy, C₃₋₁₀-cycloalkylsulphanyl, C₃₋₁₀-cycloalkylsulphinyl, C₃₋₁₀-cycloalkylsulphonyl, C₅₋₁₀-cycloalkenylsulphanyl, C₅₋₁₀-cycloalkenylsulphinyl, C₅₋₁₀-cycloalkenyl-sulphonyl, arylsulphanyl, arylsulphinyl, heteroarylsulphanyl or heteroarylsulphinyl,
while the above-mentioned alkynyl and alkenyl groups may be mono- or polysubstituted by fluorine or chlorine, and
the above-mentioned alkynyl and alkenyl groups may be mono- or disubstituted by identical or different groups L1;
while the above-mentioned cycloalkyl and cycloalkenyl rings may be mono- or disubstituted independently of one another by substituents selected from fluorine and C₁₋₃-alkyl, and
in the above-mentioned cycloalkyl and cycloalkenyl rings one or two methylene groups may be replaced independently of one another by O, S, CO, SO, SO₂ or NR^{N},
- R^{N}: denotes H, C₁₋₄-alkyl, C₁₋₄-alkylcarbonyl or C₁₋₄-alkylsulphonyl,
- L1: independently of one another are selected from among hydroxy, cyano, nitro, C₃₋₇-cycloalkyl, aryl, heteroaryl, C₁₋₄-alkylcarbonyl, arylcarbonyl, heteroarylcarbonyl, aminocarbonyl, C₁₋₄-alkylaminocarbonyl, di-(C₁₋₃-alkyl)-aminocarbonyl, pyrrolidin-1-ylcarbonyl, piperidin-1-ylcarbonyl, morpholin-4-ylcarbonyl, arylaminocarbonyl, heteroarylaminocarbonyl, C₁₋₄-alkoxycarbonyl, aryl-C₁₋₃-alkoxycarbonyl, heteroaryl-C₁₋₃-alkoxycarbonyl, C₁₋₄-alkyloxy, aryloxy, heteroaryloxy, C₁₋₄-alkylsulphanyl, arylsulphanyl, heteroarylsulphanyl, C₁₋₄-alkylsulphinyl, arylsulphinyl, heteroarylsulphinyl, C₁₋₄-alkylsulphonyl, arylsulphonyl and heteroarylsulphonyl; and
- L2: independently of one another are selected from among fluorine, chlorine, bromine, iodine, C₁₋₃-alkyl, difluoromethyl, trifluoromethyl, C₁₋₃-alkoxy, difluoromethoxy, trifluoromethoxy and cyano; and
- R⁶, R^{7a}, R^{7b}, R^{7c}: independently of one another have a meaning selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl,
while by the aryl groups mentioned in the definition of the above groups are meant phenyl or naphthyl groups which may be mono- or disubstituted independently of one another by identical or different groups L2; and
by the heteroaryl groups mentioned in the definition of the above groups are meant a pyrrolyl, furanyl, thienyl, pyridyl, indolyl, benzofuranyl, benzothiophenyl, quinolinyl, isoquinolinyl or tetrazolyl group,
or is meant a pyrrolyl, furanyl, thienyl or pyridyl group, wherein one or two methyne groups are replaced by nitrogen atoms,
or is meant an indolyl, benzofuranyl, benzothiophenyl, quinolinyl or isoquinolinyl group, wherein one to three methyne groups are replaced by nitrogen atoms,
while the above-mentioned heteroaryl groups independently of one another may be mono- or disubstituted by identical or different groups L2;
while, unless otherwise stated, the above-mentioned alkyl groups may be straight-chain or branched,
the tautomers, the stereoisomers thereof, the mixtures thereof and the salts thereof.

In another embodiment, Active Ingredient A is comprised of: Glucopyranosyl-substituted phenyl of general formula I.2 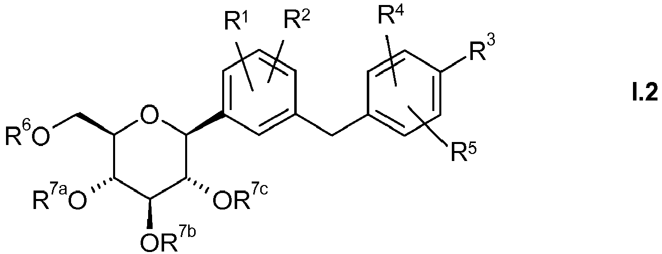 wherein the groups R¹ to R⁶ and R^{7a}, R^{7b} and R^{7c} are defined as immediately above.

More particularly, an Active Ingredient A compound can be selected from the group consisting of:
(1) 1-chloro-2-(4-cyclopentyloxybenzyl)-4-(ß-D-glucopyranos-1-yl)-benzene
(2) 1-chloro-4-(ß-D-glucopyranos-1-yl)-2-[4-(*(R)*-tetrahydrofuran-3-yloxy)-benzyl]-benzene
(3) 1-chloro-4-(ß-D-glucopyranos-1-yl)-2-[4-(*(S)*-tetrahydrofuran-3-yloxy)-benzyl]-benzene
(4) 1-chloro-4-(ß-D-glucopyranos-1-yl)-2-[4-(tetrahydrofuran-2-on-3-yloxy)-benzyl]-benzene
(5) 1-chloro-4-(ß-D-glucopyranos-1-yl)-2-(4-cyclobutyloxy-benzyl)-benzene
(6) 1-chloro-4-(ß-D-glucopyranos-1-yl)-2-(4-cyclohexyloxy-benzyl)-benzene
(7) 1-chloro-4-(ß-D-glucopyranos-1-yl)-2-[4-(tetrahydropyran-4-yloxy)-benzyl]-benzene
(8) 1-chloro-4-(ß-D-glucopyranos-1-yl)-2-[4-(1-acetyl-piperidin-4-yloxy)-benzyl]-benzene
(10) 1-(ß-D-Glucopyranos-1-yl)-4-methyl-3-[4-(tetrahydrofuran-3-yloxy)-benzyl]-benzene
(11) 1-(ß-D-Glucopyranos-1-yl)-4-methyl-3-[4-(2-trimethylsilyl-ethyl)-benzyl]-benzene
(12) 1-chloro-4-(ß-D-glucopyranos-1-yl)-2-(4-ethynyl-benzyl)-benzene
(13) 1-chloro-4-(ß-D-glucopyranos-1-yl)-2-[4-(piperidin-4-yloxy)-benzyl]-benzene
(14) 1-fluoro-4-(ß-D-glucopyranos-1-yl)-2-(4-ethynyl-benzyl)-benzene
(15) 1-(ß-D-glucopyranos-1-yl)-3-(4-ethynyl-benzyl)-benzene
(16) 1-ethynyl-4-(ß-D-glucopyranos-1-yl)-2-(4-ethoxy-benzyl)-benzene
(17) 1-methoxy-4-(ß-D-glucopyranos-1-yl)-2-(4-ethynyl-benzyl)-benzene

In another embodiment, Active Ingredient A is 1-chloro-4-(ß-D-glucopyranos-1-yl)-2-[4-(*(S)*-tetrahydrofuran-3-yloxy)-benzyl]-benzene (hereinafter referred to as "Compound 1.a") and described in the international patent application WO 2005/092877 having the chemical structure according to formula 1.a:

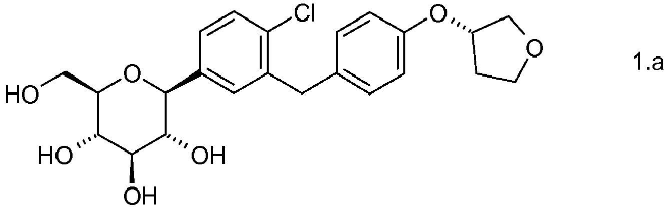

In another embodiment, Active Ingredient A is 1-chloro-2-(4-cyclopentyloxybenzyl)-4-(ß-D-glucopyranos-1-yl)-benzene (hereinafter referred to as "Compound 1.b") and described in the international patent application WO 2005/092877 having the chemical structure according to formula 1.b:

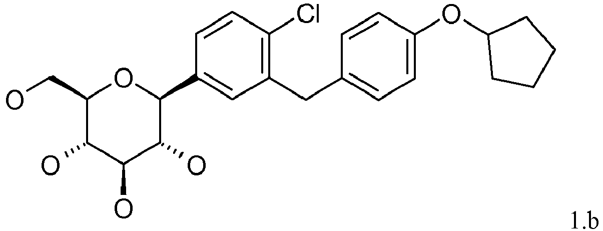

In another embodiment, Active Ingredient A is 1-chloro-4-(ß-D-glucopyranos-1-yl)-2-[4-(*(R)*-tetrahydrofuran-3-yloxy)-benzyl]-benzene (hereinafter referred to as "Compound 1.c") and described in the international patent application WO 2005/092877 having the chemical structure according to formula 1.c:

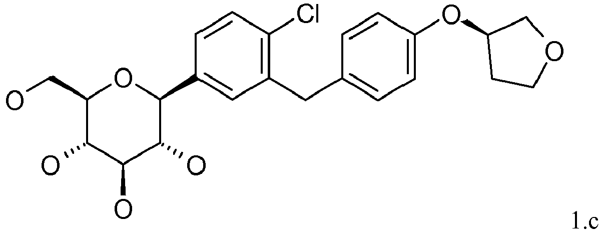

In another embodiment, Active Ingredient A is 1-(ß-D-glucopyranos-1-yl)-4-methyl-3-[4-(tetrahydrofuran-3-yloxy)-benzyl]-benzene_(hereinafter referred to as "Compound 1.d") and described in the international patent application WO 2005/092877 having the chemical structure according to formula 1.d:

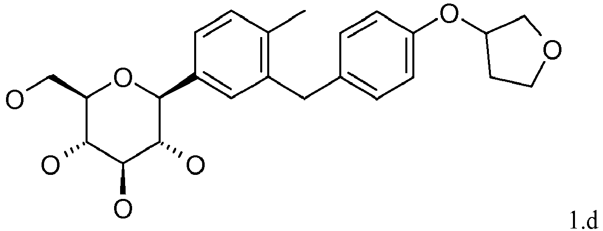

In another embodiment, Active Ingredient A is 1-chloro-4-(ß-D-glucopyranos-1-yl)-2-(4-ethynyl-benzyl)-benzene (hereinafter referred to as "Compound 1.e") and described in the international patent application WO 2005/092877 having the chemical structure according to formula 1.e:

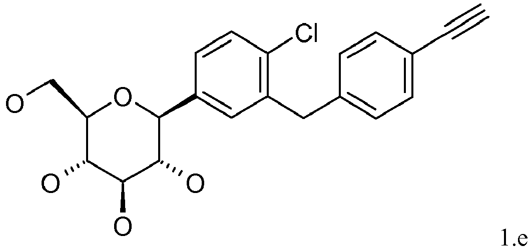

In another embodiment, Active Ingredient A is 1-fluoro-4-(ß-D-glucopyranos-1-yl)-2-(4-ethynyl-benzyl)-benzene (hereinafter referred to as "Compound 1.f") and described in the international patent application WO 2005/092877 having the chemical structure according to formula 1.f:

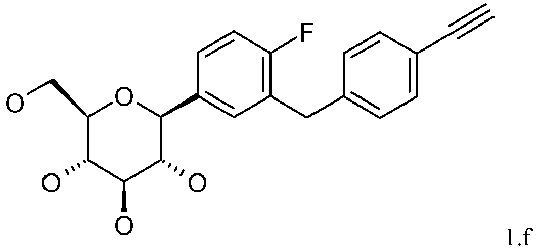

In another embodiment, Active Ingredient A is 1-ethynyl-4-(ß-D-glucopyranos-1-yl)-2-(4-ethoxy-benzyl)-benzene (hereinafter referred to as "Compound 1.g") and described in the international patent application WO 2005/092877 having the chemical structure according to formula 1.g:

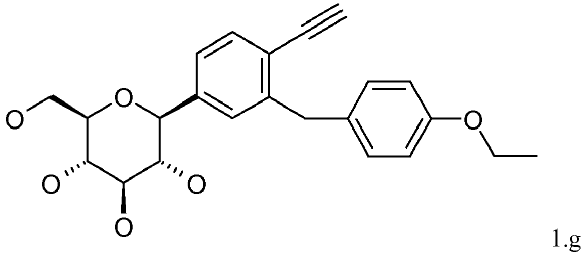

### B) Active Ingredient B Inhibitor compounds which may be used according to the invention.

Active Ingredients B compounds which are suitable for such combinations include for example those which potentiate the therapeutic effect of SGLT-2 inhibitor compounds such as Active Ingredient A compounds. Therapeutic agents which are suitable for such a combination include, for example, antidiabetic agents such as metformin, sulphonylureas (e.g. glibenclamide, tolbutamide, glymepiride), meglitinides (e.g. nateglinide, repaglinide), PPAR-gamma-agonists (e.g. rosiglitazone, pioglitazone), and antagonists (e.g. SR-202), PPAR-gamma/alpha modulators (e.g. KRP 297), alpha-glucosidase inhibitors (e.g. acarbose, voglibose).

Preferably, the Active Ingredient B compound is selected from the following groups of Active Ingredient B's consisting of:
a) biguanides,
b) sulfonylureas, (SU)
c) thiazolidinediones (TZD, PPAR gamma agonists)
d) alpha-glucosidase blockers,
e) insulin and insulin analogues,
f) GLP1 and GLP1 analogues,
g) PPAR gamma modulators including PPAR gamma antagonists and PPAR gamma partial agonists (e.g. metaglidasen),
h) PPAR gamma/alpha modulators,
i) glucose-dependent insulinotropic polypeptide agonists,
j) beta-3 agonists, and
k) glucokinase activators.

Examples of (a) biguanides are metformin, phenformin and buformin. SGLT-2 Inhibitors in combination with metformin can improve glycemic control and may act synergistically with metformin to reduce weight that has overall beneficial effects on the metabolic syndrome which is commonly associated with T2DM.

Examples of (b) sulfonylureas are glibenclamide, tolbutamide, glymepiride, glipizide, glyburide, gliclazide. As the efficacy of SUs wears off over the course of treatment, adding an SGLT-2 Inhibitor to an SU may offer additional benefit to the patient in terms of better glycemic control. Also, treatment with SUs is normally associated with gradual weight gain over the course of treatment and weight reducing capability of SGLT-2 Inhibitor that has been shown in pre-clinical studies, can minimize this side effect of the treatment with an SU and improve the metabolic syndrome. This combination may also allow a reduction in the dose of SU which may translate into less hypoglycemia which is an undesirable side effect of SUs.

Examples of (c) thiazolidindiones are pioglitazone, rosiglitazone, troglitazone and ciglitazone, especially pioglitazone and rosiglitazone. Expected additional benefits from the combination of an SGLT-2 Inhibitor and TZDs are synergistic reduction in blood glucose (better glycemic control), improvement of fluid retention caused by TZDs and nullifying weight gain associated with the use of TZDs.

Examples of (d) alpha-glucosidase blockers are miglitol, acarbose and voglibose. Combining an SGLT-2 Inhibitor to alpha-glucosidase blockers will add to their blood glucose lowering effect and may allow a reduction in the dose of the alpha-glucosidase blocker that are commonly associated with unpleasant gastro-intestinal side effects, thereby making it more tolerable and improve the patient's compliance with the treatment.

Examples of (e) insulins and insulin analogues are human insulin, insulin lispro, insulin glusilin, recombinant insulins such as insulin aspart, NPH insulin, insulin detemir, insulin zinc suspension and insulin glargin. The use of insulin is commonly associated with weight gain as a result of the anabolic effects of insulin as well as fluid retension. Combining an SGLT-2 Inhibition with insulin will achieve a better glycemic control with lower doses of insulin. Given SGLT-2 Inhibitors mechanism of action, they are likely to ameliorate the fluid retention and edema associated with insulin use.

An example of (f) GLP1 and GLP1 analogues is exendin-4 (exenatide). Combining an SGLT-2 inhibitor with a GLP-1 analogue is expected to improve glycemic control and add to GLP-1 analogue weight reducing effect.

An example of (g) PPAR gamma modulators is metaglidasen. Combining an SGLT-2 inhibitor with a PPAR gamma modulator is expected to improve glycemic control.

Examples of (h) PPAR gamma/alpha modulators are tesaglitazar, muraglitazar and KRP297. Combining an SGLT-2 inhibitor with a PPAR gamma/alpha modulator is expected to improve glycemic control.

Examples of (i) glucose-dependent insulinotropic polypeptide agonists are pramlintide and amlyin. Combining an SGLT-2 inhibitor with these compounds is expected to improve glycemic control.

Examples of (j) beta-3 agonists are ritobegron, YM 178, solabegron, talibegron, N-5984, GRC-1087, rafabegron and FMP825. Combining an SGLT-2 inhibitor with a beta-3 agonist is expected to improve glycemic control.

An example of (k) glucokinase activators, is PSN010 (OSI Pharmaceuticals). Combining an SGLT-2 inhibitor with a glucokinase activator is expected to improve glycemic control.

Other examples of suitable Active Ingredient B compounds that can be used in combination with an Active Ingredient A compounds are inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase, lipid lowering agents such as for example HMG-CoA-reductase inhibitors (e.g. simvastatin, atorvastatin), fibrates (e.g. bezafibrate, fenofibrate), nicotinic acid and the derivatives thereof, PPAR-alpha agonists, PPAR-delta agonists, ACAT inhibitors (e.g. avasimibe) or cholesterol absorption inhibitors such as, for example, ezetimibe, bile acid-binding substances such as, for example, cholestyramine, inhibitors of ileac bile acid transport, HDL-raising compounds such as CETP inhibitors or ABC1 regulators or active substances for treating obesity, such as sibutramine or tetrahydrolipostatin, orlistat, dexfenfluramine, axokine, antagonists of the cannabinoid1 receptor, MCH-1 receptor antagonists, MC4 receptor agonists, NPY5 or NPY2 antagonists or ß3-agonists such as SB-418790 or AD-9677 and agonists of the 5HT2c receptor.

### Other Aspects of the combinations

The invention also relates to pharmaceutical preparations, containing one or more Active Ingredient A and Active Ingredient B, or the pharmaceutically acceptable derivatives thereof, optionally combined with conventional excipients and/or carriers.

Any reference to the abovementioned SGLT-2 and/or Active Ingredient A and B includes any "pharmaceutically acceptable derivatives" thereof which refers to any pharmaceutically acceptable salt or ester of a compound of this invention, or any other compound which, upon administration to a patient, is capable of providing (directly or indirectly), a pharmacologically active metabolite or pharmacologically active residue thereof. A pharmacologically active metabolite shall be understood to mean any Active Ingredient A or B of the invention capable of being metabolized enzymatically or chemically.

Pharmaceutically acceptable salts of the compounds of this invention include those derived from pharmaceutically acceptable inorganic and organic acids and bases. Examples of suitable acids include hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds of this invention and their pharmaceutically acceptable acid addition salts. Salts derived from appropriate bases include alkali metal (e.g., sodium), alkaline earth metal (e.g., magnesium), ammonium and N-(C₁-C₄ alkyl)⁴⁺ salts.

In addition, the compounds of this invention include prodrugs of SGLT-2 and Active Ingredient A and B compounds. Prodrugs include those compounds that, upon simple chemical transformation, are modified to produce compounds of the invention. Simple chemical transformations include hydrolysis, oxidation and reduction. Specifically, when a prodrug of this invention is administered to a patient, the prodrug may be transformed into a compound B of the invention, thereby imparting the desired pharmacological effect.

For therapeutic use, the pharmaceutical combinations of one or more Active Ingredient A and Active Ingredient B according to the invention may be administered in any conventional dosage form in any conventional manner. Routes of administration include, but are not limited to, intravenously, intramuscularly, subcutaneously, intrasynovially, by infusion, sublingually, transdermally, orally, topically or by inhalation.
The preferred modes of administration are oral, topical or intravenous.

The pharmaceutical combinations of Active Ingredient A and Active Ingredient B according to the invention may be administered separately, or in a combination formulation with adjuvants that enhance stability of the inhibitors, facilitate administration of pharmaceutical compositions containing them in certain embodiments, provide increased dissolution or dispersion, increase inhibitory activity, provide adjunct therapy, and the like, including other active ingredients. Advantageously, such combination therapies utilize lower dosages of the conventional therapeutics, thus avoiding possible toxicity and adverse side effects incurred when those agents are used as monotherapies. Pharmaceutical combinations of Active Ingredient A and Active Ingredient B may therefore be physically combined with the conventional therapeutics or other adjuvants into a single pharmaceutical composition. The optimum percentage (w/w) of a compound of the invention may vary and is within the purview of those skilled in the art. As mentioned above, dosage forms of the compositions described herein include pharmaceutically acceptable carriers and adjuvants known to those of ordinary skill in the art. These carriers and adjuvants include, for example, ion exchangers, alumina, aluminum stearate, lecithin, serum proteins, buffer substances, water, salts or electrolytes and cellulose-based substances. Preferred dosage forms include, tablet, capsule, caplet, liquid, solution, suspension, emulsion, lozenges, syrup, reconstitutable powder, granule, suppository and transdermal patch. Methods for preparing such dosage forms are known (see, for example, H.C. Ansel and N.G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient.

Regarding Active Ingredient A, in some embodiments, dosage levels range from about 1 to 1000 mg/dose, or preferably 10 to 500mg for a 70 kg patient. Although one dose per day may be sufficient, up to 5 doses per day may be given. For oral doses, up to 2000 mg/day may be required. Reference in this regard may also be made to US20050209166. The dosage of Active Ingredient A required to achieve the corresponding activity for treatment or prevention usually depends on the compound which is to be administered, the patient, the nature and gravity of the illness or condition and the method and frequency of administration and is for the patient's doctor to decide. Expediently, the dosage may be from 1 to 100 mg, preferably 1 to 30 mg, by intravenous route, and 1 to 1000 mg, preferably 1 to 100 mg, by oral route, in each case administered 1 to 4 times a day. For this purpose, the compounds of formula I prepared according to the invention may be formulated, optionally together with other active substances, together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof, to produce conventional galenic preparations such as plain or coated tablets, capsules, powders, suspensions or suppositories.

As the skilled artisan will appreciate, lower or higher doses may be required depending on particular factors. For instance, specific dosage and treatment regimens will depend on factors such as the patient's general health profile, the severity and course of the patient's disorder or disposition thereto, and the judgment of the treating physician.

In another aspect the present invention relates to a pharmaceutical composition suitable for inhalation which contains one or more salts and one or more compounds, optionally in the form of their solvates or hydrates. The active substances may either be combined in a single preparation or contained in two separate formulations. Pharmaceutical compositions which contain the Active Substances A and B in a single preparation are preferred according to the invention.

The present invention also relates to the use of Active Ingredient A and Active Ingredient B for preparing a pharmaceutical combinations containing therapeutically effective quantities of Active Ingredient A and Active Ingredient B for treating diabetes, provided that treatment SGLT-2 inhibitors is not contraindicated from a therapeutic point of view, by simultaneous or successive administration.

In the active substance combinations of Active Ingredient A and Active Ingredient B according to the invention, ingredients A and B may be present in the form of their enantiomers, mixtures of enantiomers or in the form of racemates.

The proportions in which the two Active Ingredients A and B may be used in the active substance combinations according to the invention are variable. Active Ingredients A and B may possibly be present in the form of their solvates or hydrates. Depending on the choice of Active Ingredients A and B, the weight ratios which may be used within the scope of the present invention vary on the basis of the different molecular weights of the various compounds and their different potencies. Determination of ratios by weight is dependent on particular active ingredients of Active Ingredients A and B, and within the skill in the art.

The active substance combinations of Active Ingredients A and B according to the invention may be administered by inhalation or by nasal application. For this purpose, Active Ingredients A and B have to be made available in inhalable forms. Inhalable preparations include inhalable powders, propellant-containing metering aerosols or propellant-free inhalable solutions. Inhalable powders according to the invention containing the combination of active substances A and B may consist of the active substances on their own or of a mixture of the active substances with physiologically acceptable excipients. Within the scope of the present invention, the term propellant-free inhalable solution also includes concentrates or sterile inhalable solutions ready for use. The preparations according to the invention may contain the combination of Active Ingredients A and B either together in one formulation or in two separate formulations. These formulations which may be used within the scope of the present invention are described in more detail in the next part of the specification.

The Examples which follow serve to illustrate the present invention in more detail without restricting the scope of the invention to the following embodiments by way of example.

### Starting materials

### Active Ingredient A, SGLT-2 inhibitor:

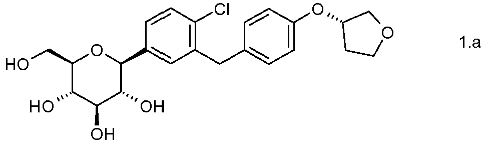

The above SGLT-2 Active Inhibitor A used in the following examples, may be obtained as described in US 11/406,971 and US Ser. No. 13/416,683.
Active Ingredient A may also be any one of Compounds 1.b, 1.c, 1.d, 1.e, 1.f or 1.g described hereinabove.

In one embodiment of the invention Active Ingredient B is selected from the group consisting of metformin, glibenclamide, tolbutamide, glymepiride, glipizid, gliquidon, glibornurid, gliclazid, nateglinide, repaglinide, pioglitazone, rosiglitazone, miglitol, insulin, metaglidasen and pramlintide.

In another embodiment of the invention at least one Active Ingredient B is selected from the group consisting of metformin, glymepiride, pioglitazone, rosiglitazone, miglitol and insulin.

Active Ingredient B can also be comprised of a biological agent, which shall be understood to mean any natural or artificial/synthetic biological molecule or fragment thereof as known in the art, such as antibodies, proteins, fusion proteins, receptors, nucleic acids, lipids, carbohydrates and the like.

Another embodiment of the invention provides for one or more Active Ingredient A in combination with one or more drugs for influencing high blood pressure, chronic heart failure or atherosclerosis such as e.g. A-II antagonists or ACE inhibitors, ECE inhibitors, diuretics, ß-blockers, Ca-antagonists, centrally acting antihypertensives, antagonists of the alpha-2-adrenergic receptor, inhibitors of neutral endopeptidase, thrombocyte aggregation inhibitors and others or combinations thereof are suitable. Examples of angiotensin II receptor antagonists are candesartan cilexetil, potassium losartan, eprosartan mesylate, valsartan, telmisartan, irbesartan, EXP-3174, L-158809, EXP-3312, olmesartan, medoxomil, tasosartan, KT-3-671, GA-0113, RU-64276, EMD-90423, BR-9701, etc. Angiotensin II receptor antagonists are preferably used for the treatment or prevention of high blood pressure and complications of diabetes, often combined with a diuretic such as hydrochlorothiazide.

Another embodiment of the invention provides of one or more Active Ingredient A compounds in combination with an Active Ingredient B compounds such as uric acid synthesis inhibitors or uricosurics is suitable for the treatment or prevention of gout.

Another embodiment of the invention provides of one or more Active Ingredient A compounds in combination with GABA-receptor antagonists, Na-channel blockers, topiramat, protein-kinase C inhibitors, advanced glycation end product inhibitors or aldose reductase inhibitors may be used for the treatment or prevention of complications of diabetes.

In general the amount of the Active Ingredient B used in the composition of the invention is usefully 1/5 of the lowest dose normally recommended up to 1/1 of the normally recommended dose.

A preferred dosage range of metformin is 100 to 3000 mg, in particular 250 to 3000 mg or 200 to 2000 mg, preferably 500 to 2000 mg, most preferably 500 to 1000 per day. The preferred range of amounts in the pharmaceutical composition for an administration once, twice or three times daily is 100 to 3000, 50 to 1500 and 35 to 1000 mg respectively. Examples are 500 or 850 mg once, twice or three times daily, 1000 mg once or twice daily or 2000 mg once daily.

A preferred dosage range of pioglitazone is 5 to 50 mg per day, especially 15 to 45 mg per day. The preferred range of amounts in the pharmaceutical composition for an administration once, twice or three times daily is 5 to 50, 2 to 25 and 2 to 20 mg respectively. Examples are 15, 30 or 45 mg once daily.

A preferred dosage range of a thiazolidindione (other than pioglitazone or rosiglitazone as described above) is 2 to 50 mg per day. The preferred range of amounts in the pharmaceutical composition for an administration once, twice or three times daily is 2 to 50, 1 to 25 and 1 to 17 mg respectively.

A preferred dosage range of miglitol is 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily.

A preferred dosage range of glibenclamide is 1 to 20 mg per day. The preferred range of amounts in the pharmaceutical composition for an administration once, twice or three times daily is 1 to 20, 0.5 to 10 and 0.5 to 7 mg respectively.

A preferred dosage range of tolbutamide is 100 to 3000 mg, preferably 500 to 3000 mg per day. The preferred range of amounts in the pharmaceutical composition for an administration once, twice or three times daily is 100 to 3000, 50 to 1500 and 35 to 1000 mg respectively.

A preferred dosage range of glymepiride is 0.5 to 10 mg, in particular 1 to 6 mg per day. The preferred range of amounts in the pharmaceutical composition for an administration once, twice or three times daily is 0.5 to 10, 0.25 to 5 and 0.2 to 3 mg respectively.

A preferred dosage range of glipizid is 1 to 50 mg, in particular 2.5 to 40 mg per day. The preferred range of amounts in the pharmaceutical composition for an administration once, twice or three times daily is 1 to 50, 0.5 to 25 and 0.3 to 17 mg respectively.

A preferred dosage range of gliquidon is 10 to 150 mg, in particular 30 to 120 mg per day. The preferred range of amounts in the pharmaceutical composition for an administration once, twice or three times daily is 10 to 150, 5 to 75 and 3 to 50 mg respectively.

A preferred dosage range of glibornurid is 5 to 75 mg per day. The preferred range of amounts in the pharmaceutical composition for an administration once, twice or three times daily is 5 to 75, 3 to 40 and 2 to 25 mg respectively.

A preferred dosage range of gliclazid is 25 to 200 mg, in particular 80 to 160 mg per day. The preferred range of amounts in the pharmaceutical composition for an administration once, twice or three times daily is 25 to 200, 12 to 100 and 10 to 70 mg respectively.

A preferred dosage range of nateglinide is 15 to 200 mg, in particular 60 to 180 mg per day.

The preferred range of amounts in the pharmaceutical composition for an administration once, twice or three times daily is 15 to 200, 7 to 100 and 5 to 70 mg respectively.

A preferred dosage range of repaglinide is 0.1 to 10 mg, in particular 0.5 to 4 mg per day. The preferred range of amounts in the pharmaceutical composition for an administration once, twice or three times daily is 0.1 to 10, 0.05 to 5 and 0.03 to 3 mg respectively.

A preferred dosage range of metaglidasen is 40 to 600 mg, in particular 200 to 600 mg per day. The preferred range of amounts in the pharmaceutical composition for an administration once, twice or three times daily is 40 to 600, 20 to 300 and 15 to 200 mg respectively.

A preferred dosage range of a PPAR gamma/alpha modulator is 0.5 to 10 mg, in particular 2.5 to 5 mg per day. The preferred range of amounts in the pharmaceutical composition for an administration once, twice or three times daily is 0.5 to 10, 0.2 to 5 and 0.1 to 3 mg respectively.

A preferred dosage range of an alpha glucosidase blocker is 0.1 to 100 mg per day. The preferred range of amounts in the pharmaceutical composition for an administration once, twice or three times daily is 0.1 to 100, 0.05 to 50 and 0.03 to 35 mg respectively.

A preferred dosage range of a pramlintide is 15 µg to 120 µg per day. The preferred range of amounts in the pharmaceutical composition for an administration once, twice or three times daily is 15 to 120, 8 to 60 and 5 to 40 µg respectively.

A preferred dosage range of a insulin is 1 to 250 IU per day. The preferred range of amounts in the pharmaceutical composition for an administration once, twice or three times daily is 1 to 250, 0.5 to 125 and 0.3 to 90 IU respectively. The term "IU" means insulin units.

The use of the Active Ingredients according to the invention, or a physiologically acceptable salt thereof, in combination with another active substance may take place simultaneously or at staggered times, but particularly within a short space of time. If they are administered simultaneously, the two active substances are given to the patient together; while if they are used at staggered times the two active substances are given to the patient within a period of less than or equal to 12 hours, but particularly less than or equal to 6 hours.

The combination of Active Ingredient compounds according to the invention, and physiologically acceptable salts thereof, may both be present together in one formulation, for example a tablet or capsule, or separately in two identical or different formulations, for example as a so-called kit-of-parts.

The Examples that follow are intended to illustrate the present invention without restricting it:

### FORMULATIONS

### I. Examples of Pharmaceutical Formulation of Active Ingredient A

### Example A

### Tablets containing 100 mg of active substance

### Composition:

**1 tablet contains:**

| | |
|---|---|
| Active Ingredient A (SGLT-2 Inhitior) | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

### Method of Preparation:

The active substance, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.
Weight of tablet: 220 mg
Diameter: 10 mm, biplanar, facetted on both sides and notched on one side.

### Example B

### Tablets containing 150 mg of active substance

### Composition:

**1 tablet contains:**

| | |
|---|---|
| Active Ingredient A (SGLT-2 Inhibitor) | 150.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

### Preparation:

The active substance mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.
Weight of tablet: 300 mg
die: 10 mm, flat

### Example C

### Hard gelatine capsules containing 150 mg of active substance

### Composition:

**1 capsule contains:**

| | | |
|---|---|---|
| Active Ingredient A | | 150.0 mg |
| corn starch (dried) | approx. | 180.0 mg |
| lactose (powdered) | approx. | 87.0 mg |
| magnesium stearate | 3.0 mg | |
| | approx. | 420.0 mg |

### Preparation:

The active substance is mixed with the excipients, passed through a screen with a mesh size of 0.75 mm and homogeneously mixed using a suitable apparatus. The finished mixture is packed into size 1 hard gelatine capsules.
Capsule filling: approx. 320 mg
Capsule shell: size 1 hard gelatine capsule.

### Example D

### Suppositories containing 150 mg of active substance

### Composition:

**1 suppository contains:**

| | |
|---|---|
| Active Ingredient A | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2,000.0 mg |

### Preparation:

After the suppository mass has been melted the active substance is homogeneously distributed therein and the melt is poured into chilled moulds.

### Example E

### Ampoules containing 10 mg active substance

### Composition:

| | |
|---|---|
| Active Ingredient A | 10.0 mg |
| 0.01 N hydrochloric acid q.s. | |
| double-distilled water ad | 2.0 ml |

### Preparation:

The active substance is dissolved in the necessary amount of 0.01 N HCl, made isotonic with common salt, filtered sterile and transferred into 2 ml ampoules.

### Example F

### Ampoules containing 50 mg of active substance

### Composition:

| | |
|---|---|
| Active Ingredient A | 50.0 mg |
| 0.01 N hydrochloric acid q.s. | |
| double-distilled water ad | 10.0 ml |

### Preparation:

The active substance is dissolved in the necessary amount of 0.01 N HCl, made isotonic with common salt, filtered sterile and transferred into 10 ml ampoules.

### III. Specific Examples of Pharmaceutical Combinations of A and B

1)

| Ingredients | dosage |
|---|---|
| Active Ingredient A: | 1 to 1000 mg per day, preferably 10 to 500 mg per day, for example 2.5 to 200 mg per day or 10 to 50 mg per day. |
| 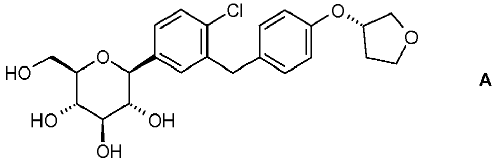 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

2)

| Ingredients | dose |
|---|---|
| Active Ingredient A : | 1 to 1000 mg daily, preferably 10 to 500 mg per day, for example 2.5 to 200 mg per day or 10 to 50 mg per day. |
| 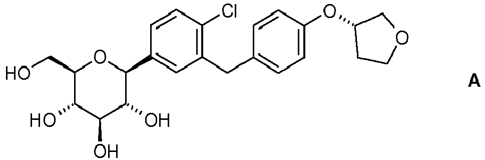 | |
| Active Ingredient B: | 2 mg, 4 mg or 8 mg per day. |
| b) glymepiride | |

3)

| Ingredients | Dosage |
|---|---|
| Active Ingredient A: | 1mg to 1000mg per day, preferably 10 to 500 mg per day, for example 2.5 to 200 mg per day or 10 to 50 mg per day. |
| 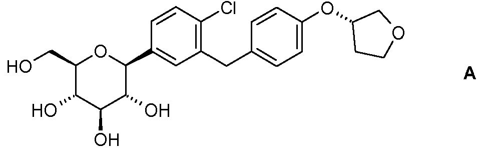 | |
| Active Ingredient B | 2 to 50 mg per day. Administration once twiece or three times daily at 2 to 50, 1 to 25 and 1 to 17 mg respectively. |
| c) thiazolidindones | |

4)

| Ingredients | dose |
|---|---|
| Active Ingredient A: | 1 to 1000 mg daily, preferably 10 to 500 mg per day, for example 2.5 to 200 mg per day or 10 to 50 mg per day. |
| 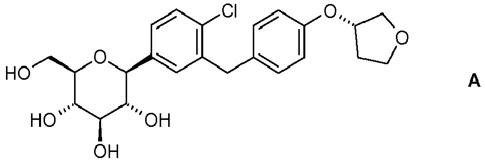 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

5)

| Ingredients | dosage |
|---|---|
| Active Ingredient A: | 1 to 1000 mg per day, preferably 10 to 500 mg per day. |
| 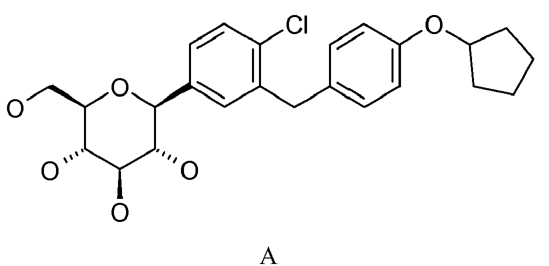 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

6)

| Ingredients | dose |
|---|---|
| Active Ingredient A : | 1 to 1000 mg daily, preferably 10 to 500 mg per day. |
| 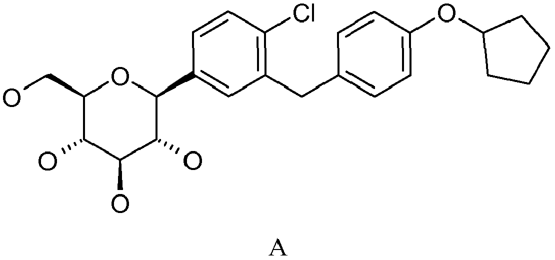 | |
| Active Ingredient B: | 2 mg, 4 mg or 8 mg per day. |
| b) glymepiride | |

7)

| Ingredients | Dosage |
|---|---|
| Active Ingredient A: | 1mg to 1000mg per day, preferably 10 to 500 mg per day. |
| 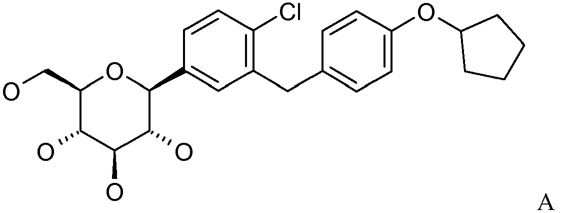 | |
| Active Ingredient B | 2 to 50 mg per day. |
| c) thiazolidindones | Administration once twiece or three times daily at 2 to 50, 1 to 25 and 1 to 17 mg respectively. |

8)

| Ingredients | dose |
|---|---|
| Active Ingredient A: | 1 to 1000 mg daily, preferably 10 to 500 mg per day. |
| 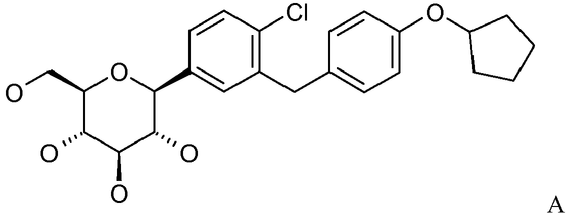 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

9)

| Ingredients | dosage |
|---|---|
| Active Ingredient A: | 1 to 1000 mg per day, preferably 10 to 500 mg per day. |
| 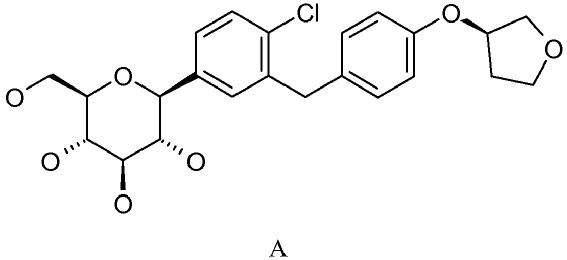 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

10)

| Ingredients | dose |
|---|---|
| Active Ingredient A : | 1 to 1000 mg daily, preferably 10 to 500 mg per day. |
| 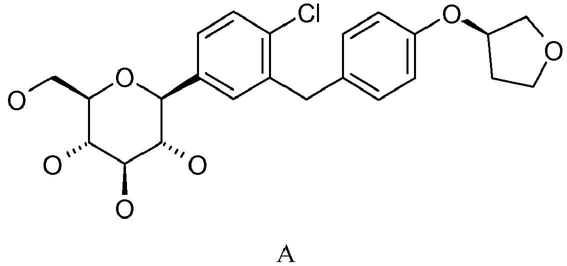 | |
| Active Ingredient B: | 2 mg, 4 mg or 8 mg per day. |
| b) glymepiride | |

11)

| Ingredients | Dosage |
|---|---|
| Active Ingredient A: | 1mg to 1000mg per day, preferably 10 to 500 mg per day. |
| 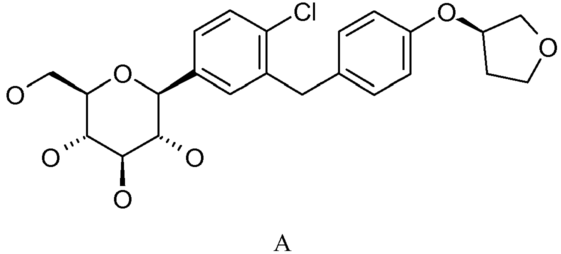 | |
| Active Ingredient B | 2 to 50 mg per day. |
| c) thiazolidindones | Administration once twiece or three times daily at 2 to 50, 1 to 25 and 1 to 17 mg respectively. |

12)

| Ingredients | dose |
|---|---|
| Active Ingredient A: | 1 to 1000 mg daily, preferably |
| 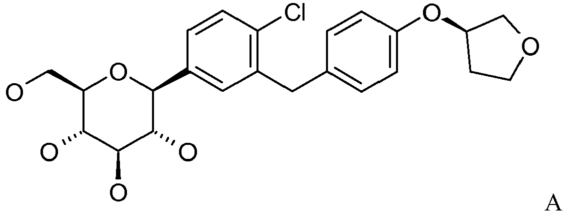 | 10 to 500 mg per day. |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

13)

| Ingredients | dosage |
|---|---|
| Active Ingredient A: | 1 to 1000 mg per day, preferably 10 to 500 mg per day. |
| 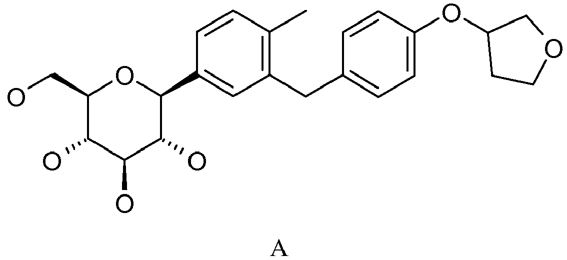 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

14)

| Ingredients | dose |
|---|---|
| Active Ingredient A : | 1 to 1000 mg daily, preferably 10 to 500 mg per day. |
| 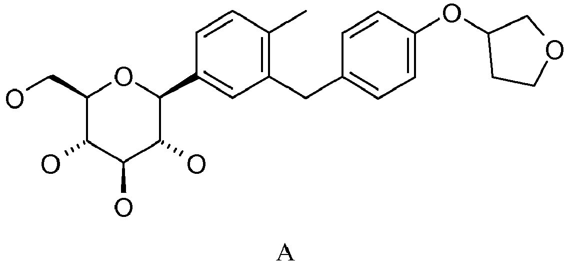 | |
| Active Ingredient B: | 2 mg, 4 mg or 8 mg per day. |
| b) glymepiride | |

15)

| Ingredients | Dosage |
|---|---|
| Active Ingredient A: | 1mg to 1000mg per day, preferably 10 to 500 mg per day. |
| 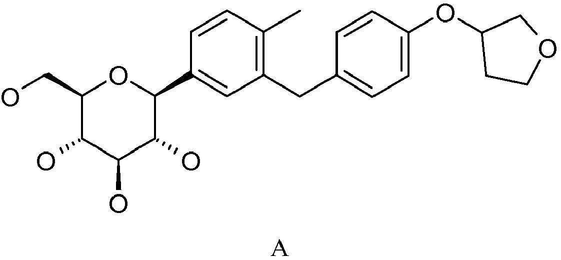 | |
| Active Ingredient B | 2 to 50 mg per day. Administration once twiece or three times daily at 2 to 50, 1 to 25 and 1 to 17 mg respectively. |
| c) thiazolidindones | |

16)

| Ingredients | dose |
|---|---|
| Active Ingredient A: | 1 to 1000 mg daily, preferably 10 to 500 mg per day. |
| 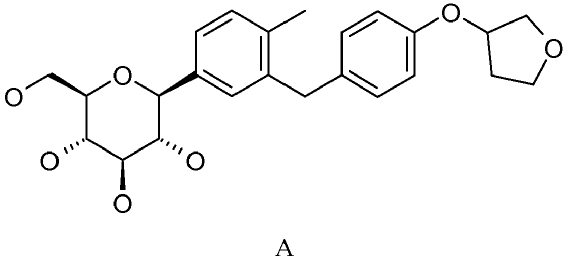 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

17)

| Ingredients | dosage |
|---|---|
| Active Ingredient A: | 1 to 1000 mg per day, preferably 10 to 500 mg per day. |
| 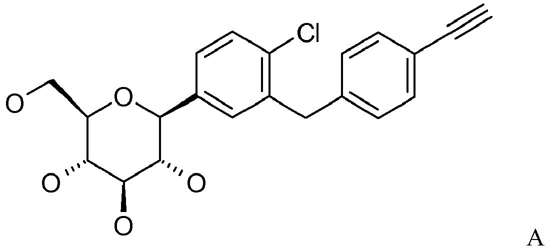 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

18)

| Ingredients | dose |
|---|---|
| Active Ingredient A : | 1 to 1000 mg daily, preferably 10 to 500 mg per day. |
| 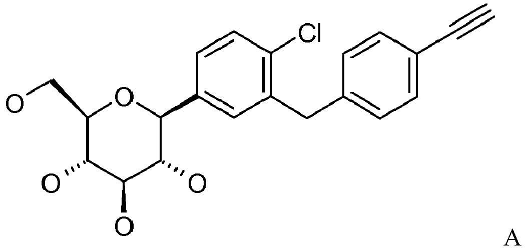 | |
| Active Ingredient B: | 2 mg, 4 mg or 8 mg per day. |
| b) glymepiride | |

19)

| Ingredients | Dosage |
|---|---|
| Active Ingredient A: | 1mg to 1000mg per day, preferably 10 to 500 mg per day. |
| 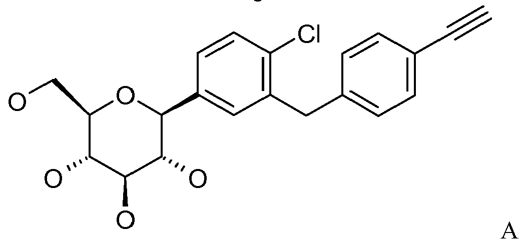 | |
| Active Ingredient B | 2 to 50 mg per day. |
| c) thiazolidindones | Administration once twiece or three times daily at 2 to 50, 1 to 25 and 1 to 17 mg respectively. |

20)

| Ingredients | dose |
|---|---|
| Active Ingredient A: | 1 to 1000 mg daily, preferably 10 to 500 mg per day. |
| 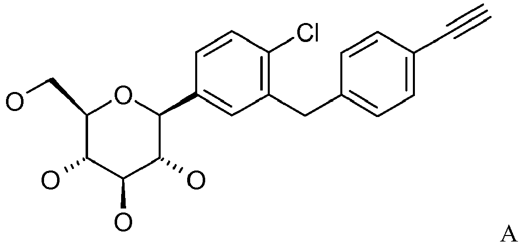 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

21)

| Ingredients | dosage |
|---|---|
| Active Ingredient A: | 1 to 1000 mg per day, preferably 10 to 500 mg per day. |
| 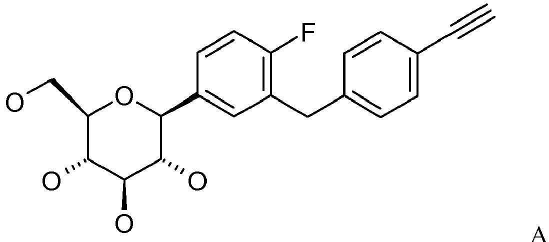 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

22)

| Ingredients | dose |
|---|---|
| Active Ingredient A : | 1 to 1000 mg daily, preferably 10 to 500 mg per day. |
| 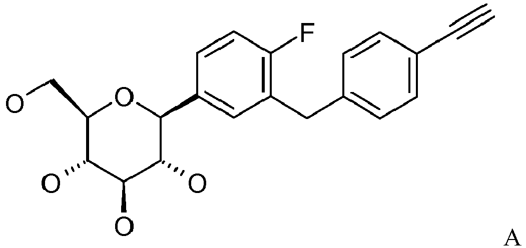 | |
| Active Ingredient B: | 2 mg, 4 mg or 8 mg per day. |
| b) glymepiride | |

23)

| Ingredients | Dosage |
|---|---|
| Active Ingredient A: | 1mg to 1000mg per day, preferably 10 to 500 mg per day. |
| 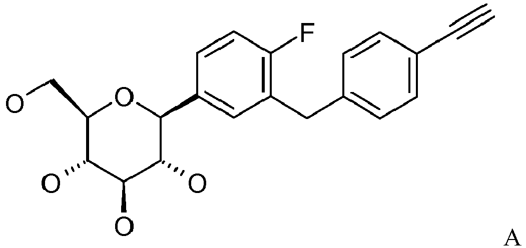 | |
| Active Ingredient B | 2 to 50 mg per day. |
| c) thiazolidindones | Administration once twiece or three times daily at 2 to 50, 1 to 25 and 1 to 17 mg respectively. |

24)

| Ingredients | dose |
|---|---|
| Active Ingredient A: | 1 to 1000 mg daily, preferably 10 to 500 mg per day. |
| 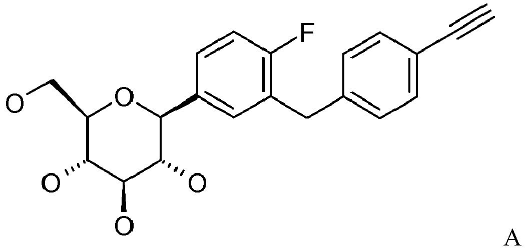 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

25)

| Ingredients | dosage |
|---|---|
| Active Ingredient A: | 1 to 1000 mg per day, preferably 10 to 500 mg per day. |
| 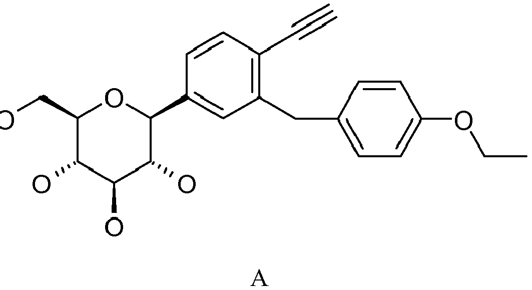 | |
| Active Ingredient B: | A preferred dosage range of 500 to 1000 mg per day, once, twice or three times daily. Examples are 500 or 850 mg once, twice or three times daily, 1000 mg once or twice daily or 2000 mg once daily. |
| a) metformin | |

26)

| Ingredients | dose |
|---|---|
| Active Ingredient A : | 1 to 1000 mg daily, preferably 10 to 500 mg per day. |
| 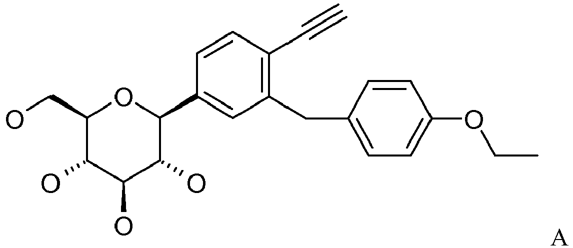 | |
| Active Ingredient B: | 2 mg, 4 mg or 8 mg per day. |
| b) glymepiride | |

27)

| Ingredients | Dosage |
|---|---|
| Active Ingredient A: | 1mg to 1000mg per day, preferably 10 to 500 mg per day. |
| 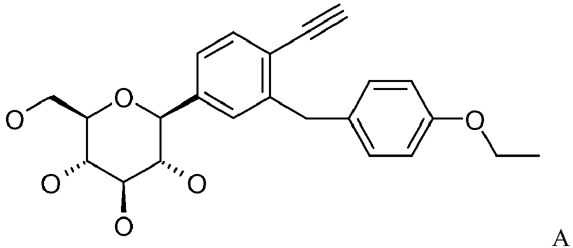 | |
| Active Ingredient B | 2 to 50 mg per day. |
| c) thiazolidindones | Administration once twiece or three times daily at 2 to 50, 1 to 25 and 1 to 17 mg respectively. |

28)

| Ingredients | dose |
|---|---|
| Active Ingredient A: | 1 to 1000 mg daily, preferably 10 to 500 mg per day. |
| 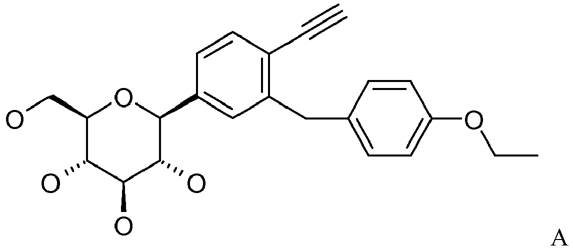 | |
| Active Ingredient B | 10 to 300 mg per day. The preferred range of amounts in the pharmaceutical composition for an administration once, twice or three times daily is 10 to 300, 5 to 150 and 3 to 100 mg respectively. Examples are 50 or 100 mg once, twice or three times daily. |
| d) miglitol | |

Other formulations comprising particular Active Ingredient A and B can be obtained based on the teachings and the examples provided herein, and from materials and methods known in the art without undue experimentation. These variations are within the scope of the invention.

### ANIMAL MODELS:

Any of the above mentioned combinations within the scope of the invention may be tested by animal models known in the art. In the following *in vivo* experiments are described which are suitable to evaluate pharmacologically relevant properties of pharmaceutical compositions and methods according to this invention.

Pharmaceutical compositions and methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control of the combinations according to this invention can be tested after single dosing of a SGLT-2 inhibitor compound(s) and a second therapeutic agent(s) which is suitable for the treatment of metabolic disorders alone and in combination in an oral glucose tolerance test in the animal models described hereinbefore. The time course of blood glucose is followed after on oral glucose challenge in overnight fasted animals. The combinations according to the present invention significantly improve glucose excursion compared to each monotherapy as measured by reduction of peak glucose concentrations or reduction of glucose AUC. In addition, after multiple dosing of a SGLT-2 inhibitor compound(s) and a second therapeutic agent(s) which is suitable for the treatment of metabolic disorders alone and in combination in the animal models described hereinbefore, the effect on glycemic control can be determined by measuring the HbA1c value in blood.

The possible dose reduction of either the SGLT-2 inhibitor compound(s) and the second therapeutic agent(s) which is suitable for the treatment of metabolic disorders or of both active ingredients can be tested by the effect on glycemic control of lower doses of the combinations and monotherapies in the animal models described hereinbefore.

The improved independence from insulin of the treatment according to this invention can be shown after single dosing in oral glucose tolerance tests in the animal models described hereinbefore. The time course of plasma insulin is followed after a glucose challenge in overnight fasted animals.

The increase in active GLP-1 levels by treatment according to this invention after single or multiple dosing can be determined by measuring those levels in the plasma of animal models described hereinbefore in either the fasting or postprandial state. Likewise, a reduction in glucagon levels in plasma can be measured under the same conditions.

An effect of the combination of a SGLT-2 inhibitor compound(s) and a second therapeutic agent(s) which is suitable for the treatment of metabolic disorders on beta-cell regeneration and neogenesis can be determined after multiple dosing in the animal models described hereinbefore by measuring the increase in pancreatic insulin content, or by measuring increased beta-cell mass by morphometric analysis after immunhistochemical staining of pancreatic sections, or by measuring increased glucose-stimulated insulin secretion in isolated pancreatic islets.

## Claims

1. A pharmaceutical composition comprised of one or more SGLT-2 inhibitor compound(s) in combination with one or more second therapeutic agent(s) which is suitable for the treatment of one or more metabolic disorder(s), wherein
i) said one or more SGLT-2 inhibitor compound(s) comprises a glucopyranosyl-substituted benzene derivative selected from the group of compounds 1-17:
(1) 1-chloro-2-(4-cyclopentyloxybenzyl)-4-(B-D-glucopyranos-1-yl)-benzene;
(2) 1-chloro-4-(ß-D-glucopyranos-1-yl)-2-[4-((*R*)-tetrahydrofuran-3-yloxy)-benzyl]-benzene;
(3) 1-chloro-4-(13-D-glucopyranos-1-yl)-2-[4-((S)-tetrahydrofuran-3-yloxy)-benzyl]-benzene;
(4) 1-chloro-4-(ß-D-glucopyranos-1-yl)-2-[4-(tetrahydrofuran-2-on-3-yloxy)-benzyl]-benzene;
(5) 1-chloro-4-(ß-D-glucopyranos-1-yl)-2-(4-cyclobutyloxy-benzyl)-benzene;
(6) 1-chloro-4-(ß-D-glucopyranos-1-yl)-2-(4-cyclohexyloxy-benzyl)-benzene;
(7) 1-chloro-4-(ß-D-glucopyranos-1-yl)-2-[4-(tetrahydropyran-4-yloxy)-benzyl]-benzene;
(8) 1-chloro-4-(ß-D-glucopyranos-1-yl)-2-[4-(1-acetyl-piperidin-4-yloxy)-benzyl]-benzene;
(10) 1-(ß-D-Glucopyranos-1-yl)-4-methyl-3-[4-(tetrahydrofuran-3-yloxy)-benzyl]-benzene;
(11) 1-(ß-D-Glucopyranos-1-yl)-4-methyl-3-[4-(2-trimethylsilyl-ethyl)-benzyl]-benzene;
(12) 1-chloro-4-(ß-D-glucopyranos-1-yl)-2-(4-ethynyl-benzyl)-benzene;
(13) 1-chloro-4-(ß-D-glucopyranos-1-yl)-2-[4-(piperidin-4-yloxy)-benzyl]-benzene;
(14) 1-fluoro-4-(ß-D-glucopyranos-1-yl)-2-(4-ethynyl-benzyl)-benzene;
(15) 1-(ß-D-glucopyranos-1-yl)-3-(4-ethynyl-benzyl)-benzene;
(16) 1-ethynyl-4-(ß-D-glucopyranos-1-yl)-2-(4-ethoxy-benzyl)-benzene; and
(17) 1-methoxy-4-(ß-D-glucopyranos-1-yl)-2-(4-ethynyl-benzyl)-benzene,
or the tautomers, the stereoisomers thereof, the mixtures thereof or the salts thereof;
or a prodrug thereof wherein one or more hydroxyl groups of the β-D-glucopyranosyl group are acylated with groups selected from (C₁₋₃-alkyl)carbonyl, (C₁₋₆-alkyl)oxycarbonyl, phenylcarbonyl, phenyl-(C₁₋₃-alkyl)-carbonyl, phenyloxycarbonyl and phenyl-(C₁₋₃-alkyl)-oxycarbonyl or a pharmaceutically acceptable salt thereof;
and
ii) said one or more second therapeutic agent(s) which is suitable for the treatment of one or more metabolic disorder(s) comprises:
a) a biguanide,
b) a sulfonylurea (SU),
c) a thiazolidinedione (PPAR gamma agonist),
d) an alpha-glucosidase blocker,
e) insulin or a insulin analogue,
f) GLP1 or a GLP1 analogue,
g) a PPAR gamma modulator,
h) a PPAR gamma/alpha modulator,
i) a glucose-dependent insulinotropic polypeptide agonist,
j) a beta-3 agonist, or
k) a glucokinase activator.

2. The pharmaceutical composition according to claim 1, wherin said metabolic disorder is selected from the group consisting of type 1 diabetes mellitus, type 2 diabetes mellitus, impaired glucose tolerance, hyperglycemia, postprandial hyperglycemia, overweight, obesity, including class I obesity, class II obesity, class III obesity, visceral obesity and abdominal obesity, and metabolic syndrome.

3. The pharmaceutical composition according to any one of claims 1 to 2, wherein said one or more second therapeutic agent(s) is suitable for the treatment or prevention of one or more metabolic disorder(s) selected from type 1 diabetes mellitus, type 2 diabetes mellitus, impaired glucose tolerance and hyperglycemia.

4. The pharmaceutical composition according to any one of claims 1 to 3, wherein said one or more SGLT-2 inhibitor compound(s) and said one or more second therapeutic agent(s) are administered in a single pharmaceutical composition or administered individually.

5. The pharmaceutical composition according to any one of claims 1 to 3, wherein the composition is suitable for combined or simultaneous or sequential use of the one or more SGLT-2 inhibitor compound(s) and the one or more second therapeutic agent(s).

6. The pharmaceutical composition according to any one of claims 1 to 3, wherein the one or more SGLT-2 inhibitor compound(s) and the one or more second therapeutic agent(s) are present in a single dosage form.

7. The pharmaceutical composition accord according to any one of claims 1 to 3, wherein the one or more SGLT-2 inhibitor compound(s) and the one or more second therapeutic agent(s) are present each in a separate dosage form.

8. The pharmaceutical composition according to any one of claims 1 to 7, wherein said second therapeutic agent is metformin, glymepiride, a thiazolidinedione or miglitol.

9. A method for treating or preventing a metabolic disorder comprising administering to a patient in need thereof a pharmaceutical composition according to any one of claims 1 to 8.

10. The method according to claim 9, wherein said metabolic disorder is selected from the group consisting of type 1 diabetes mellitus, type 2 diabetes mellitus, impaired glucose tolerance, hyperglycemia, postprandial hyperglycemia, overweight, obesity, including class I obesity, class II obesity, class III obesity, visceral obesity and abdominal obesity, and metabolic syndrome.

11. The method according to claim 10, wherein said one or more SGLT-2 inhibitor compound(s) and said one or more second therapeutic agent(s) are administered in a single pharmaceutical composition or administered individually.

12. The method according to any one of claims 9 to 11, wherein said second therapeutic agent is metformin, glymepiride, a thiazolidinedione or miglitol.

13. Use of a pharmaceutical composition according to any one of claims 1 to 8 for the manufacture of a medicament for use in a method according to any one of claims 9 to 12.

14. Use of a glucopyranosyl-substituted benzene derivative selected from the group of compounds 1-17 according to claim 1 for the manufacture of a medicament for use in a method according to any one of claims 9 to 12.

15. Use of a second therapeutic agent according to claim 1 or 8 for the manufacture of a medicament for use in a method according to any one of claims 9 to 12.
